# EUROPEAN PATENT APPLICATION

(11) **EP 1 254 662 A2**
(43) Date of publication of application: **06.11.2002**
(21) Application number: 02252391.4
(22) Date of filing: 02.04.2002
(51) Int. Cl.: A61K 31/445, A61K 31/55, A61P 25/18, A61P 25/22, A61P 25/24

(54) **Methods and kits for treating depression or preventing deterioration of cognitive function**

(30) Priority: 25.04.2001 US 286433 P
(71) Applicant: Pfizer Products Inc., Groton, Connecticut 06340 (US)
(72) Inventor: Day, Wesley Warren, Groton, Connecticut 06340 (US); Lee, Andrew George, Groton, Connecticut 06340 (US); Petrie, Charles David, Groton, Connecticut 06340 (US); Thompson, David Duane, Groton, Connecticut 06340 (US)
(74) Representative: Sewell, Richard Charles

(57) **Abstract**

The present invention provides methods and kits for treating depression, perimenopausal depression, schizophrenia, anxiety, panic attacks, binge eating, social phobia, or preventing deterioration of cognitive function by administering to a patient in need thereof a therapeutically effect amount of an estrogen agonist /antagonist of formula I

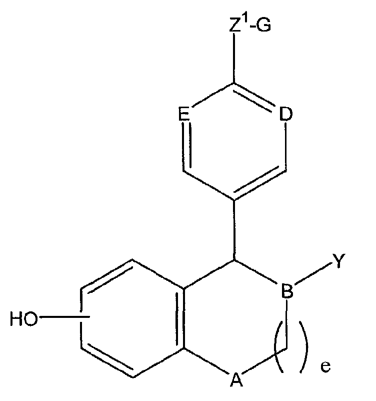

## Description

### Field of the Invention

The present invention relates to the use of an estrogen agonist / antagonist for treating depression, perimenopausal depression, schizophrenia, anxiety, panic attacks, binge eating, social phobia, or preventing deterioration of cognitive function.

### Background of the Invention

Estrogen has been associated with affective disorders. Depression is an affective disorder in which a patient feels sadness of such a scope and/or duration as to be clinically distinguishable from normal sadness. Depressed patients can have an overwhelming sense of uselessness and can feel lethargic and possibly suicidal. Unlike normal depression due to causative factors such as a death or bad news, a patient with clinical depression is unable to adjust to the causative factors over time and can remain in the depressed state for long periods of time. Other types of affective disorders can occur at particular time periods in a patient's life. For example, perimenopausal depression can occur in women who are near menopause.

Other disorders in which estrogen has been associated are psychiatric disorders. Examples of psychiatric disorders are schizophrenia, anxiety, panic attacks, binge eating and social phobia.

Estrogen has also been associated with cognitive functioning such as the ability to learn, long and short term memory, verbal and visual memory, recall, and visual reproduction. In addition, estrogen has been associated with memory loss such as in Alzheimer's disease and senile dementia.

Treating affective disorders, psychiatric disorders or preventing deterioration of cognitive function has been a goal of recent medical research. The present invention provides methods and kits for treating depression, perimenopausal depression, schizophrenia, anxiety, panic attacks, binge eating, social phobia, or preventing deterioration of cognitive function by administering to a patient in need thereof a therapeutically effect amount of an estrogen agonist / antagonist.

### Summary of the Invention

The present invention provides methods of treating depression or perimenopausal depression, the methods comprising administering to a patient in need thereof a therapeutically effective amount of an estrogen agonist / antagonist of formula I 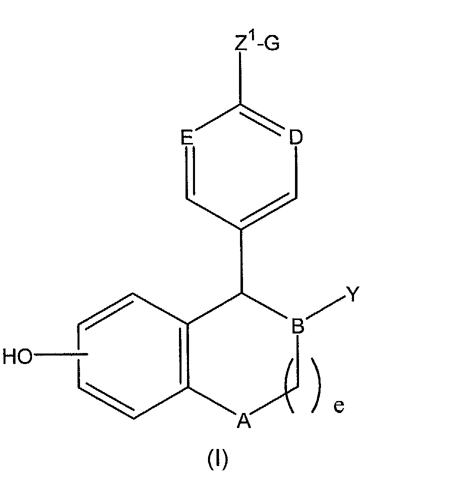 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d)-OCHR₂CHR³-;or
   (e)-SCHR²CHR³-;
G is
   (a) -NR⁷R⁸;
   (b) 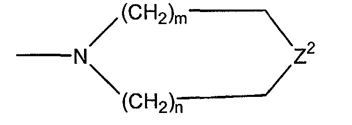 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 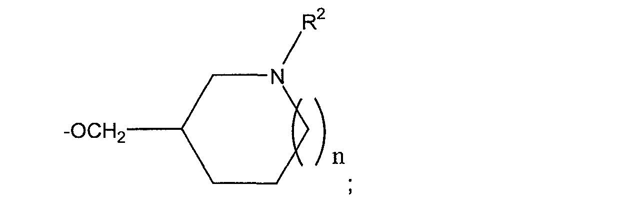
W is
   (a) -CH₂-;
   (b) -CH=CH-;
   (c) -O-;
   (d) -NR²-;
   (e) -S(O)ₙ-;
   (f) 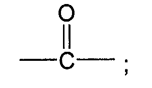
   (g) -CR²(OH)-;
   (h) -CONR²-;
   (i) -NR²CO-;
   (j) 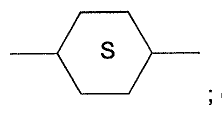 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
   a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   or an optical or geometric isomer thereof; or a pharmaceutically acceptable salt, N-oxide, ester, quaternary ammonium salt or prodrug thereof.

In a preferred embodiment of the methods, the estrogen agonist / antagonist is a compound of formula (IA) 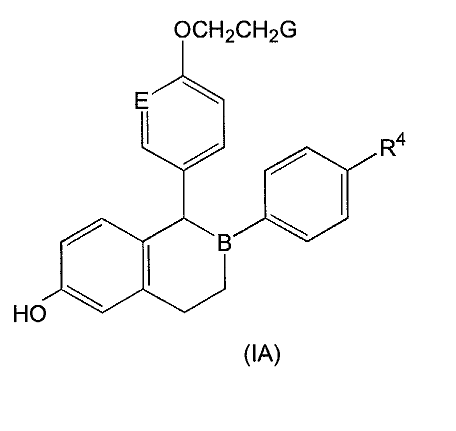 wherein G is

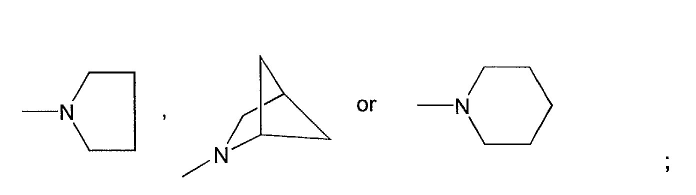

R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In another preferred embodiment of the methods, the estrogen agonist /antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In another preferred embodiment of the methods, the estrogen agonist /antagonist is in the form of a D-tartrate salt.

In another preferred embodiment of the methods, an additional compound that is useful to treat depression or perimenopausal depression is administered to the patient.

In another preferred embodiment of the methods with an additional compound, the additional compound is a selective serotonin reuptake inhibitor.

In another preferred embodiment of the methods wherein the additional compound is a selective serotonin reuptake inhibitor, the selective serotonin reuptake inhibitor is sertraline (Zoloft®), paroxetine (Paxil®), fluoxetine (Prozac®) or citalopram (Celexa®) or a pharmaceutically acceptable salt or prodrug thereof or salt of a prodrug of the selective serotonin reuptake inhibitor.

Also provided by the present invention are methods of treating schizophrenia, anxiety, panic attacks, binge eating or social phobia, the methods comprising administering to a patient in need thereof a therapeutically effective amount of an estrogen agonist / antagonist of Formula I 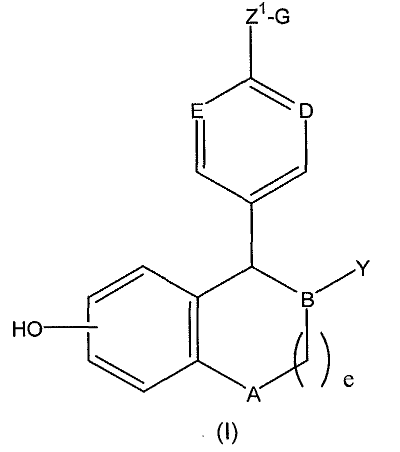 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 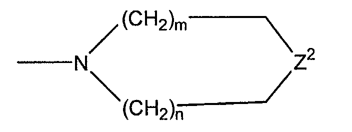 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 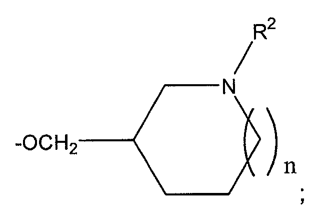
W is
   (a) -CH₂-;
   (b) -CH=CH-;
   (c) -O-;
   (d) -NR²-;
   (e) -S(O)ₙ-;
   (f) 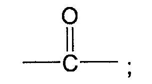
   (g) -CR²(OH)-;
   (h) -CONR²-;
   (i) -NR²CO-;
   (j) 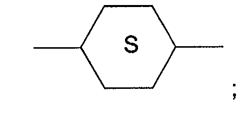 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   or an optical or geometric isomer thereof; or a pharmaceutically acceptable salt, N-oxide, ester, quaternary ammonium salt or prodrug thereof.

In a preferred embodiment of the methods, the estrogen agonist /antagonist is a compound of formula (IA) 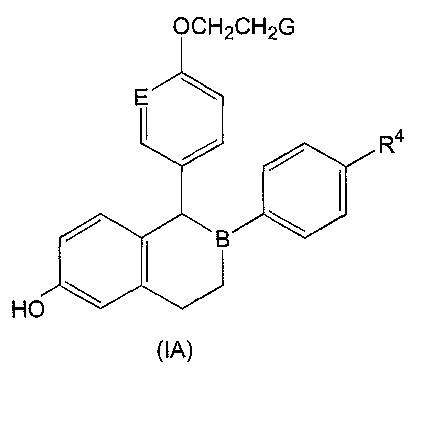 wherein G is

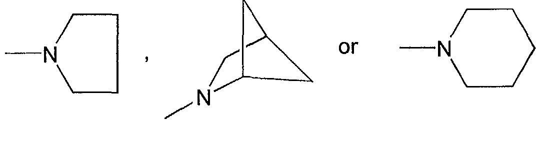

R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In another preferred embodiment of the methods, the estrogen agonist /antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In another preferred embodiment of the methods, the estrogen agonist /antagonist is in the form of a D-tartrate salt.

In another preferred embodiment of the methods, an additional compound that is useful to treat schizophrenia, anxiety, panic attacks, binge eating or social phobia is administered to the patient.

Also provided by the present invention are methods of preventing deterioration of cognitive function, the methods comprising administering to a patient in need thereof a therapeutically effective amount of an estrogen agonist / antagonist of Formula I 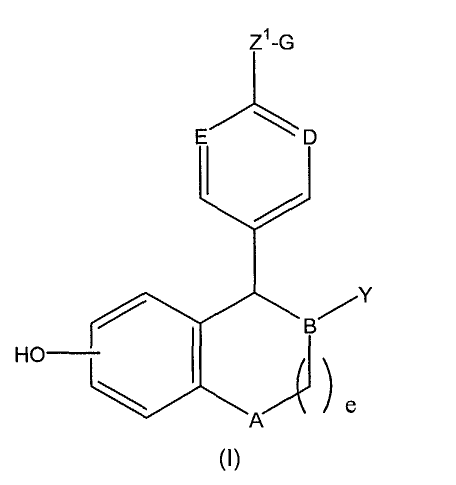 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 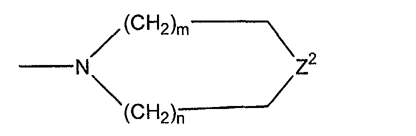 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 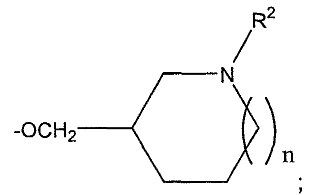
W is
   (a) -CH₂-;
   (b) -CH=CH-;
   (c) -O-;
   (d) -NR²-;
   (e) -S(O)ₙ-;
   (f) 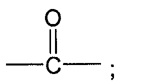
   (g) -CR²(OH)-;
   (h) -CONR²-;
   (i) -NR²CO-;
   (j) 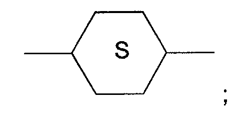 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   or an optical or geometric isomer thereof; or a pharmaceutically acceptable salt, N-oxide, ester, quaternary ammonium salt or prodrug thereof.

In a preferred embodiment of the methods, the estrogen agonist /antagonist is a compound of formula (IA) 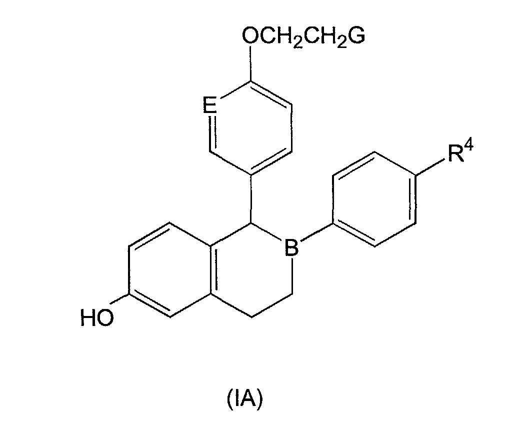 wherein G is

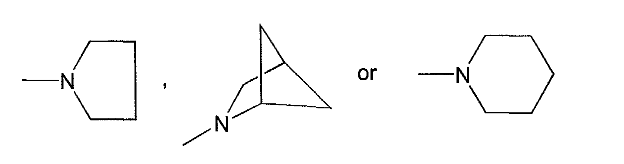

R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In another preferred embodiment of the methods, the estrogen agonist /antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In another preferred embodiment of the methods, the estrogen agonist /antagonist is in the form of a D-tartrate salt.

In another preferred embodiment of the methods, an additional compound that is useful to prevent deterioration of cognitive function is administered to the patient.

Also provided by the present invention are kits for use to treat depression, perimenopausal depression, schizophrenia, anxiety, panic attacks, binge eating, social phobia, or prevent deterioration of cognitive function, the kits comprising:
(A) a pharmaceutical composition comprising an estrogen agonist /antagonist of formula I 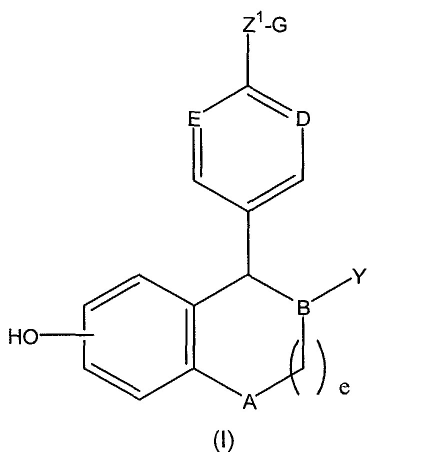
wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 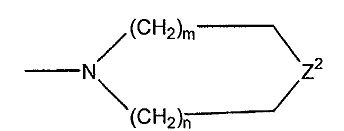 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 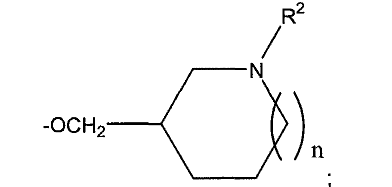
W is
   (a) -CH₂-;
   (b) -CH=CH-;
   (c) -O-;
   (d) -NR²-;
   (e) -S(O)ₙ-;
   (f) 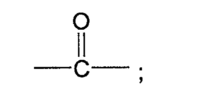 or
   (g) -CR²(OH)-;
   (h) -CONR²-;
   (i) -NR²CO-;
   (j) 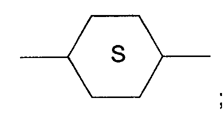 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   or an optical or geometric isomer thereof; or a pharmaceutically acceptable salt, N-oxide, ester, quaternary ammonium salt or prodrug thereof; and
   (B) instructions describing a method of using the pharmaceutical composition to treat depression, perimenopausal depression, schizophrenia, anxiety, panic attacks, binge eating, social phobia, or prevent the deterioration of cognitive function.

In a preferred embodiment of the kits, the estrogen agonist / antagonist is a compound of formula (IA) 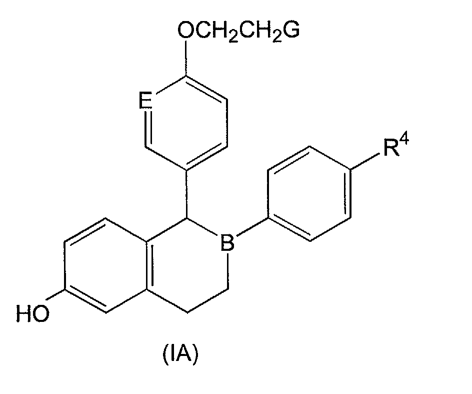 wherein G is

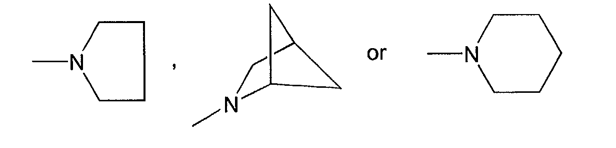

R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

In another preferred embodiment of the kits, the estrogen agonist /antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

In another preferred embodiment of the kits, the estrogen agonist /antagonist is in the form of a D-tartrate salt.

In another preferred embodiment, the kits include an additional compound that is useful to treat depression, perimenopausal depression, schizophrenia, anxiety, panic attacks, binge eating, social phobia, or prevent deterioration of cognitive function.

Also provided are pharmaceutical compositions comprising a compound of Formula I and one or more compounds selected from sertraline, donepezil, tacrine, or ziprasidone, or a pharmaceutically acceptable salt or prodrug thereof, or a salt of a prodrug.

In a preferred embodiment of the pharmaceutical compositions the compound of Formula I is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

### Detailed Description of the Invention

The present invention provides methods and kits for treating depression, perimenopausal depression, schizophrenia, anxiety, panic attacks, binge eating, social phobia, or preventing deterioration of cognitive function by administering to a patient in need thereof a therapeutically effect amount of an estrogen agonist /antagonist.

The terms "treat", "treatment", and "treating" include preventative (e.g., prophylactic) and palliative treatment or the act of providing preventative or palliative treatment.

The term "patient" means animals, particularly mammals. Preferred patients are humans. Particularly preferred patients are postmenopausal women.

The term "postmenopausal women" includes not only women of advanced age who have passed through menopause, but also women who have been hysterectomized or for some other reason have suppressed estrogen production, such as those who have undergone long-term administration of corticosteroids, suffer from Cushings' syndrome, or have gonadal dysgenesis.

An "estrogen agonist / antagonist" is a compound that affects some of the same receptors that estrogen does, but not all, and in some instances, it antagonizes or blocks estrogen. It is also known as a "selective estrogen receptor modulator" (SERM). Estrogen agonists / antagonists may also be referred to as antiestrogens although they have some estrogenic activity at some estrogen receptors. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 1973;22:707, Capony et al., Mol Cell Endocrinol, 1975;3:233).

One facet of cognitive function relates to learning and memory. The ability to learn relates a patient's capacity to acquire, retain or generalize specific skills or sets of information. A patient's ability to learn can be affected by deficiencies in attention, memory, perception or reasoning.

One aspect of learning is memory. Two primary types of memory have been defined: long term and short term. Other aspects of memory include recall and recognition. Disorders relating to memory include problems with long term memory, short term memory, and verbal and visual memory.

Cognitive function, in addition to memory, also includes reasoning, such as abstract reasoning; perception; and spatial orientation.

The phrase "preventing deterioration of cognitive function" includes the prevention of deterioration of one of more of the following cognitive domains: orientation, attention and concentration, psychomotor speed and function, language and naming, verbal memory (immediate and delayed recall), category fluency, abstract reasoning and praxis (motor integration and executive control of complex learned movements). The prevention of deterioration of cognitive function can include prevention in patients not yet showing deterioration of cognitive function, and preferably, in patients who have shown deterioration in cognitive function.

Anxiety can be classified into several varieties including generalized anxiety disorder, anxiety associated with a medical condition, acute stress disorder, post traumatic stress disorder, social anxiety disorder, specific anxiety disorder, and the like. The various types of anxiety are well known to those skilled in the art and can be treated in accordance with the present invention. The various types of anxiety are described in detail in *Diagnostic and Statistical Manual of Mental Disorders*, Fourth Edition, American Psychiatric Association, Washington, D.C., 1994, which is also known as the DSM IV.

The estrogen agonists / antagonists of the present invention may be administered systemically or locally. For systemic use, the estrogen agonists /antagonists herein are formulated for parenteral (e.g., intravenous, subcutaneous, intramuscular, intraperitoneal, intranasal or transdermal) or enteral (e.g., oral or rectal) delivery according to conventional methods. Intravenous administration can be by a series of injections or by continuous infusion over an extended period. Administration by injection or other routes of discretely spaced administration can be performed at intervals ranging from weekly to once to three or more times daily.

Preferred estrogen agonists / antagonists of the present invention include the compounds described in U.S. Patent 5,552,412. Those compounds are described by the formula designated herein as formula (I) given below: 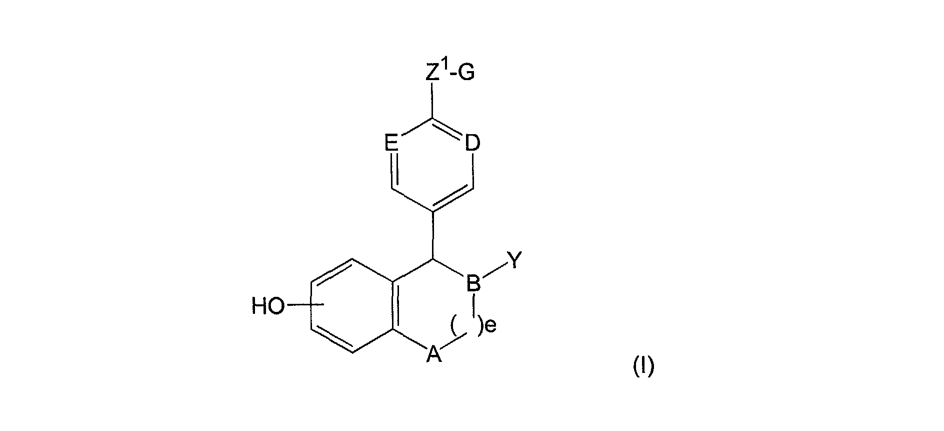 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 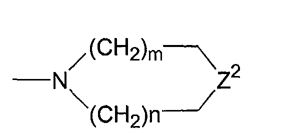 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 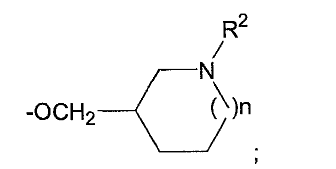
W is
   (a) -CH₂-;
   (b) -CH=CH-;
   (c) -O-;
   (d) -NR²-;
   (e) -S(O)ₙ-;
   (f) 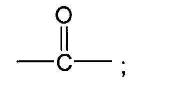
   (g) -CR²(OH)-;
   (h) -CONR²-;
   (i) -NR²CO-;
   (j) 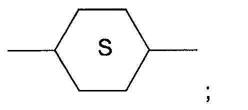 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (I) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
   e is 0, 1 or 2;
   m is 1, 2 or 3;
   n is 0, 1 or 2;
   p is 0, 1, 2 or 3;
   q is 0, 1, 2 or 3;
   and optical and geometric isomers thereof; and nontoxic pharmaceutically acceptable acid addition salts, N-oxides, esters, quaternary ammonium salts and prodrugs thereof.

Additional preferred compounds also disclosed in U.S. Patent 5,552,412 are described by the formula designated herein as formula (IA): 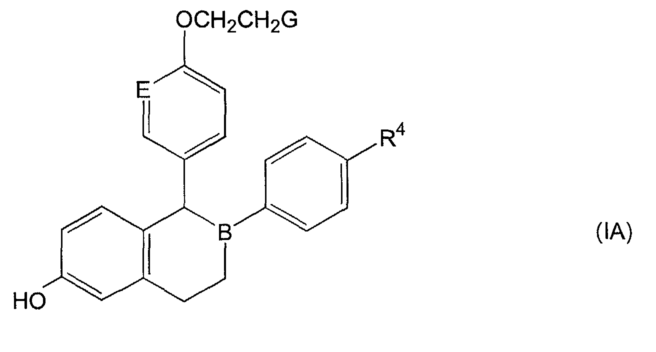 wherein G is

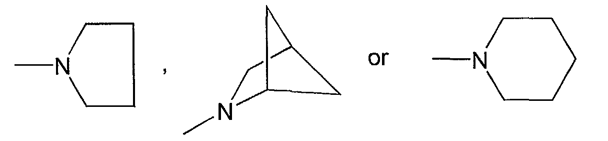

R⁴ is H, OH, F, or Cl; and B and E are independently selected from CH and N and optical and geometric isomers thereof; and nontoxic pharmaceutically acceptable acid addition salts, N-oxides, esters, quaternary ammonium salts and prodrugs thereof.

Especially preferred compounds for the methods and kits of the invention are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydro-naphthalene-2-ol;
cis-6-phenyl-5-[4-(2-pyrrolidin-1-yl-ethoxy)-phenyl]-5,6,7,8-tetrahydro-naphthalene-2-ol;
cis-1-[6'-pyrrolidinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;
1 -(4'-pyrrolidinoethoxyphenyl)-2-phenyl-6-hydroxy-1,2,3,4-tetrahydroisoquinoline; and pharmaceutically acceptable salts thereof. An especially preferred salt of (-)-cis-6-phenyl-5-[4-(2-pyrrolidin-1-yl-ethoxy)-phenyl]-5,6,7,8-tetrahydro-naphthalene-2-ol is the D-tartrate salt.

Other preferred estrogen agonists / antagonists are disclosed in U.S. Patent 5,047,431. These compounds are described by the formula designated herein as formula (II) below: 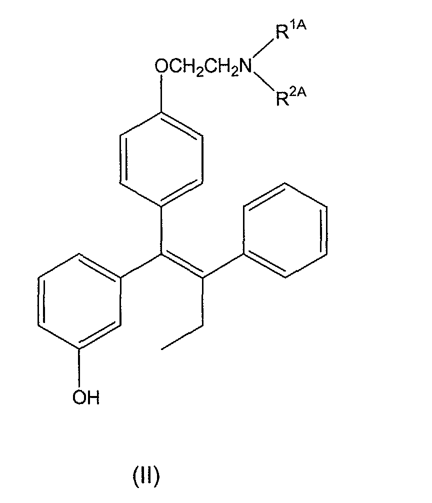 wherein

R^{1A} and R^{2A} may be the same or different and are either H, methyl, ethyl or a benzyl group; and optical or geometric isomers thereof; and pharmaceutically acceptable salts, N-oxides, esters, quaternary ammonium salts, and prodrugs. A preferred compound of formula II is droloxifene.

Additional estrogen agonists / antagonists that can be used in the methods and kits described herein are tamoxifen: (ethanamine,2-[-4-(1,2-diphenyl-1-butenyl)phenoxy]-N,N-dimethyl, (Z)-2-, 2-hydroxy-1,2,3-propanetricarboxylate(1:1)) and other compounds disclosed in U.S. Patent 4,536,516; 4-hydroxy tamoxifen (i.e., tamoxifen wherein the 2-phenyl moiety has a hydroxy group at the 4 position) and other compounds disclosed in U.S. Patent 4,623,660; raloxifene: (methanone, [6-hydroxy-2-(4-hydroxyphenyl)benzo[b]thien-3-yl][4-[2-(1-piperidinyl)ethoxy]phenyl]-,hydrochloride) and other compounds disclosed in U.S. Patents 4,418,068; 5,393,763; 5,457,117; 5,478,847 and 5,641,790; toremifene: (ethanamine, 2-[4-(4-chloro-1,2-diphenyl-1-butenyl)phenoxy]-N,N-dimethyl-, (Z)-, 2-hydroxy-1,2,3-propanetricarboxylate (1:1) and other compounds disclosed in U.S. Patents 4,696,949 and 4,996,225; centchroman: 1-[2-[[4-(-methoxy-2,2, dimethyl-3-phenyl-chroman-4-yl)-phenoxy]-ethyl]-pyrrolidine and other compounds disclosed in U.S. Patent 3,822,287; idoxifene: pyrrolidine, 1-[-[4-[[1-(4-iodophenyl)-2-phenyl-1-butenyl]phenoxy]ethyl] and other compounds disclosed in U.S. Patent 4,839,155; 6-(4-hydroxy-phenyl)-5-[4-(2-piperidin-1-yl-ethoxy)-benzyl]-naphthalen -2-ol and other compounds disclosed in U.S. Patent 5,484,795; and {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone and other compounds disclosed in published international patent application WO 95/10513. Other preferred compounds include GW 5638 and GW 7604. The synthesis of GW 5638 and GW 7604 is described in Willson et al., J. Med. Chem., 1994;37:1550-1552.

Further preferred estrogen agonists / antagonists include EM-652 (shown in the formula designated herein as formula (III)) and EM-800 (shown in the formula designated herein as formula (IV)). The synthesis of EM-652 and EM-800 and the activity of various enantiomers is described in Gauthier et al., J. Med. Chem., 1997;40:2117-2122.

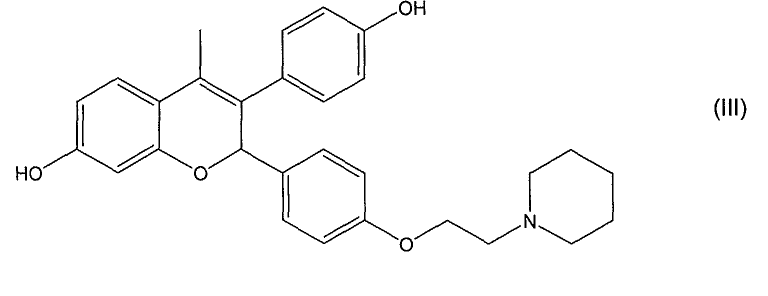

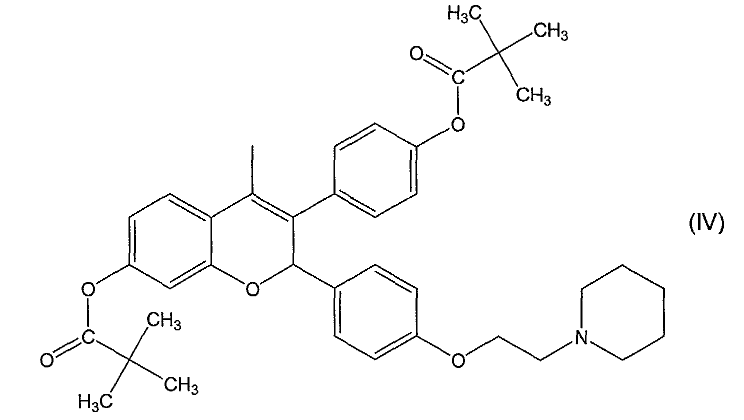

Further preferred estrogen agonists / antagonists include TSE-424 and other compounds disclosed in U.S. Patent 5,998,402, U.S. Patent 5,985,910, U.S. Patent 5,780,497, U.S. Patent 5,880,137, and European Patent Application EP 0802183 A1 including the compounds described by the formulas designated herein as formulas V and VI, below: 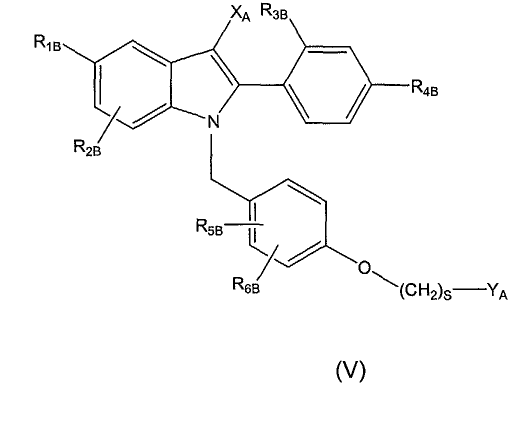 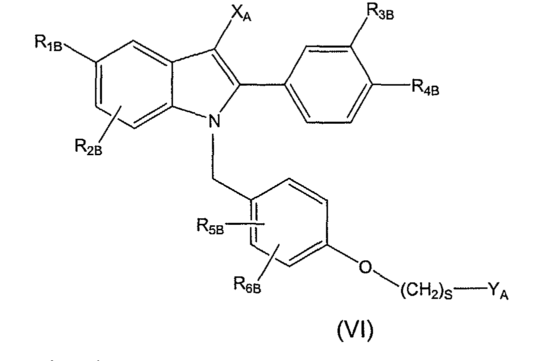 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including trifluoromethyl ether and trichloromethyl ether.
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including trifluoromethyl ether and trichloromethyl ether, cyano, C₁-C₆ alkyl (straight chain or branched), or trifluoromethyl;
X_{A} is selected from H, C₁-C₆ alkyl, cyano, nitro, trifluoromethyl, and halogen;
s is 2 or 3;
Y_{A} is selected from:
   a) the moiety: 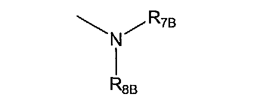 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
   b) a five-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
   e) a bicyclic heterocycle containing from 6-12 carbon atoms either bridged or fused and containing up to two heteroatoms selected from the group consisting of -O-, -NH-, -N(C₁-C₄ alkyl)-, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -N=, C₁-C₄ alkylamino, di(C₁-C₄)alkylamino, -NHSO₂R_{1B}, -NHCOR_{1B}, -NO₂, and phenyl optionally substituted with 1-3 (C₁-C₄) alkyl; and optical and geometric isomers thereof; and nontoxic pharmaceutically acceptable acid addition salts, N-oxides, esters, quaternary ammonium salts, and prodrugs thereof.

Preferred compounds are those having the general structures V or VI, above, wherein:
R_{1B} is selected from H, OH or the C₁-C₁₂ esters or alkyl ethers thereof, and halogen;
R_{2B}, R_{3B}, R_{4B}, R_{5B}, and R_{6B} are independently selected from H, OH or the C₁-C₁₂ esters or alkyl ethers thereof, halogen, cyano, C₁-C₆ alkyl, or trihalomethyl, preferably trifluoromethyl, with the proviso that, when R_{1B} is H, R_{2B} is not OH;
X_{A} is selected from H, C₁-C₆ alkyl, cyano, nitro, trifluoromethyl, and halogen;
Y_{A} is the moiety:

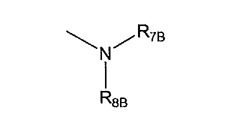

R_{7B} and R_{8B} are selected independently from H, C₁-C₆ alkyl, or combined by -(CH₂)_{w}-, wherein w is an integer of from 2 to 6, so as to form a ring, the ring being optionally substituted by up to three substituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄alkyl), -NH₂, C₁-C₄ alkylamino, C₁-C₄ dialkylamino, -NHSO₂(C₁-C₄alkyl), -CO(C₁-C₄alkyl), and -NO₂; and optical and geometric isomers thereof; and pharmaceutically acceptable salts, N-oxides, esters, quaternary ammonium salts, and prodrugs thereof.

The rings formed by a concatenated R_{7B} and R_{8B}, mentioned above, may include, but are not limited to, aziridine, azetidine, pyrrolidine, piperidine, hexamethyleneamine or heptamethyleneamine rings.

The preferred compounds of structural formulas V and VI, above, are those wherein R_{1B} is OH; R_{2B} - R_{6B} are as defined above; X_{A} is selected from the group of Cl, NO₂, CN, CF₃, or CH₃; Y_{A} is the moiety 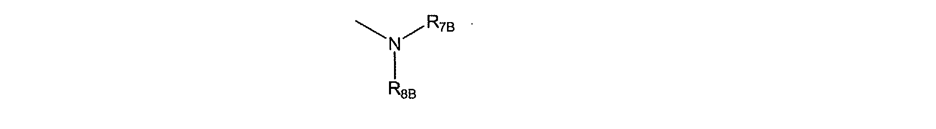 and R_{7B} and R_{8B} are concatenated together as -(CH₂)ₜ-, wherein t is an integer of from 4 to 6, to form a ring optionally substituted by up to three subsituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄)alkyl, -NH₂, C₁-C₄ alkylamino, di(C₁-C₄)alkylamino, -NHSO₂(C₁-C₄)alkyl, -NHCO(C₁-C₄)alkyl, and -NO₂; and optical and geometric isomers thereof; and pharmaceutically acceptable salts, N-oxides, esters, quaternary ammonium salts, and prodrugs thereof.

Another preferred compound is TSE-424 as described by the formula designated herein as formula (Va) below:

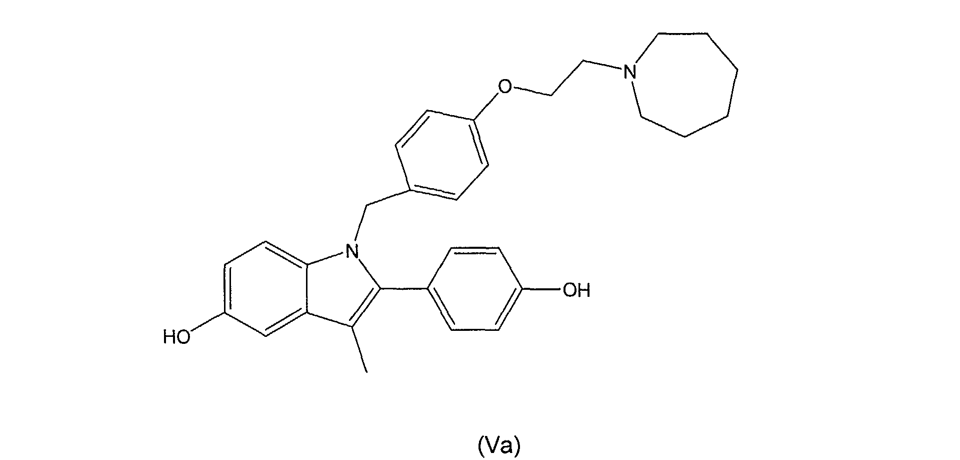

The expression "pharmaceutically acceptable salts" includes both pharmaceutically acceptable acid addition salts and pharmaceutically acceptable cationic salts. The expression "pharmaceutically-acceptable cationic salts" is intended to include, but is not limited to, such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to include, but is not limited to, such salts as the hydrochloride, hydrobromide, sulfate, hydrogen sulfate, phosphate, hydrogen phosphate, dihydrogenphosphate, acetate, succinate, citrate, tartrate, methanesulfonate (mesylate) and p-toluenesulfonate (tosylate) salts.

The pharmaceutically acceptable acid addition salts of the estrogen agonists / antagonists of this invention may be formed of the compound itself, or of any of its esters, and include the pharmaceutically acceptable salts which are often used in pharmaceutical chemistry. For example, salts may be formed with inorganic or organic acids such as hydrochloric acid, tartaric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferably with hydrochloric acid, citric acid, benzoic acid, maleic acid, acetic acid or propionic acid.

The salts of basic compounds can be formed by reacting the compound with a suitable acid. The salts are typically formed in high yields at moderate temperatures, and often are prepared by isolating the compound from a suitable acidic wash as the final step of the synthesis. The salt-forming acid is dissolved in an appropriate organic solvent, or aqueous organic solvent, such as an alkanol, ketone or ester. On the other hand, if the compound of this invention is desired in the free base form, it can be isolated from a basic final wash step. A preferred technique for preparing hydrochlorides is to dissolve the free base in a suitable solvent and dry the solution thoroughly, as over molecular sieves, before bubbling hydrogen chloride gas through it. A preferred salt of (-)-cis-6-phenyl-5-[4-(2-pyrrolidin-1-yl-ethoxy)-phenyl]-5,6,7,8-tetrahydro-naphthalene-2-ol is the D-(-)-tartrate salt (See, U.S. Patent 5,948,809). It will also be recognized that it is possible to administer amorphous forms of the estrogen agonists / antagonists.

One of ordinary skill in the art will recognize that certain estrogen agonists /antagonists of this invention will contain one or more atoms which may be in a particular stereochemical, tautomeric, or geometric configuration, giving rise to stereoisomers, tautomers and configurational isomers. All such tautomers and isomers and mixtures thereof are included in this invention. Hydrates and solvates of the compounds of this invention are also included.

The subject invention also includes isotopically-labeled estrogen agonists /antagonists, which are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds and of said prodrugs which contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Those of ordinary skill in the art will recognize that physiologically active compounds which have accessible hydroxy groups can be administered in the form of pharmaceutically acceptable esters. The compounds of this invention can be effectively administered as an ester, formed on the hydroxy groups. It is possible to adjust the rate or duration of action of the compound by appropriate choices of ester groups.

Certain ester groups are preferred when a compound of this invention contains an ester. The estrogen agonists / antagonists including the compounds of formula I, IA, II, III, IV, V, Va, or VI may contain ester groups at various positions as defined herein above, where these groups are represented as -COOR⁹, R⁹ is C₁ -C₁₄ alkyl, C₁ -C₃ chloroalkyl, C₁ -C₃ fluoroalkyl, C₅ -C₇ cycloalkyl, phenyl, or phenyl mono- or disubstituted independently with C₁ -C₄ alkyl, C₁ -C₄ alkoxy, hydroxy, nitro, chloro, fluoro or tri(chloro or fluoro)methyl.

As used herein, the term "effective amount" means an amount of compound that is capable of treating the described condition. The specific dose of a compound administered according to this invention will, of course, be determined by the particular circumstances surrounding the case including, for example, the compound administered, the route of administration and the severity of the condition being treated.

The dose of a compound of this invention to be administered to a subject is rather widely variable and subject to the judgement of the attending physician. It should be noted that it may be necessary to adjust the dose of a compound when it is administered in the form of a salt, such as a laureate, the salt forming moiety of which has an appreciable molecular weight.

The following dosage amounts are for an average human subject having a weight of about 65 kg to about 70 kg. The skilled practitioner will readily be able to determine the dosage amount required for a subject whose weight falls outside the 65 kg to 70 kg range, based upon the medical history of the subject. All doses set forth herein, and in the appendant claims, are daily doses of the free base form of the estrogen agonists / antagonists. Calculation of the dosage amount for other forms of the free base form such as salts or hydrates is easily accomplished by performing a simple ratio relative to the molecular weights of the species involved.

The general range of effective administration rates of the estrogen agonists / antagonists is from about 0.001 mg/day to about 200 mg/day. A preferred rate range is from about 0.010 mg/day to about 100 mg/day. Of course, it is often practical to administer the daily dose of compound in portions, at various hours of the day. However, in any given case, the amount of compound administered will depend on such factors as the potency of the specific estrogen agonist/antagonist, the solubility of the compound, the formulation used and the route of administration.

Methods of formulation are well known in the art and are disclosed, for example, in Remington: The Science and Practice of Pharmacy, Alphonso R. Genaro, Mack Publishing Company, Easton, Pa., 19th Edition (1995). Pharmaceutical compositions for use within the present invention can be in the form of sterile, non-pyrogenic liquid solutions or suspensions, coated capsules, suppositories, lyophilized powders, transdermal patches or other forms known in the art.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which facilitate the disintegration of a tablet to release a compound when the tablet becomes wet. They include starches, clays, celluloses, algins and gums, more particularly, corn and potato starches, methylcellulose, agar, bentonite, wood cellulose, powdered natural sponge, cation-exchange resins, alginic acid, guar gum, citrus pulp and carboxymethylcellulose, for example, may be used as well as sodium lauryl sulfate.

Tablets are often coated with sugar as a flavorant and sealant, or with film-forming protecting agents to modify the dissolution properties of the tablet. The compounds may also be formulated as chewable tablets, by using large amounts of pleasant-tasting substances such as mannitol in the formulation, as is now well-established in the art.

When it is desired to administer a compound as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the compounds may be delayed or prolonged by proper formulation. For example, a slowly soluble pellet of the compound may be prepared and incorporated in a tablet or capsule. The technique may also include making pellets of several different dissolution rates and filling capsules with a mixture of the pellets. Tablets or capsules may be coated with a film which resists dissolution for a predictable period of time. Topical formulations may be designed to yield delayed and/or prolonged percutaneous absorption of a compound. Even the parenteral preparations may be made long-acting, by dissolving or suspending the compound in oily or emulsified vehicles which allow it to disperse slowly in the serum.

The term "prodrug" means a compound that is transformed *in vivo* to yield a compound of the present invention. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

For example, if a compound of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound of the present invention comprises an amine functional group, a prodrug can be formed by the replacement of a hydrogen atom in the amine group with a group such as R^{X}-carbonyl, R^{X}O-carbonyl, NR^{X}R^{X}'-carbonyl where R^{X} and R^{X}' are each independently (C₁-C₁₀)alkyl, (C₃-C₇)cycloalkyl, benzyl, or R^{X}-carbonyl is a natural α-aminoacyl or natural α-aminoacyl-natural α-aminoacyl, -C(OH)C(O)OY^{X} wherein Y^{X} is H, (C₁-C₆)alkyl or benzyl, -C(OY^{X0}) Y^{X1} wherein Y^{X0} is (C₁-C₄) alkyl and Y^{X1} is (C₁-C₆)alkyl, carboxy(C₁-C₆)alkyl, amino(C₁-C₄)alkyl or mono-N- or di-N,N-(C₁-C₆)alkylaminoalkyl, -C(Y^{X2}) Y^{X3} wherein Y^{X2} is H or methyl and Y^{X3} is mono-N- or di-N,N-(C₁-C₆)alkylamino, morpholino, piperidin-1-yl or pyrrolidin-1-yl.

Advantageously, the present invention also provides kits for use to treat depression, perimenopausal depression, schizophrenia, anxiety, panic attacks, binge eating, social phobia, or prevent deterioration of cognitive function. The kits comprise: a) a pharmaceutical composition comprising an estrogen agonist /antagonist as specified herein; and b) instructions describing a method of using the pharmaceutical composition to treat depression, perimenopausal depression, schizophrenia, anxiety, panic attacks, binge eating, social phobia, or prevent deterioration of cognitive function.

A "kit" as used in the instant application includes a container for containing the pharmaceutical compositions and may also include divided containers such as a divided bottle or a divided foil packet. The container can be in any conventional shape or form as known in the art which is made of a pharmaceutically acceptable material, for example a paper or cardboard box, a glass or plastic bottle or jar, a resealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to market a single dosage form. For example, tablets may be contained in a bottle, which is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a written memory aid, where the written memory aid is of the type containing information and/or instructions for the physician, pharmacist or subject, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday,"... etc .... "Second Week, Monday, Tuesday, ..." etc. Other variations of memory aids will be readily apparent. A "daily dose" can be a single tablet or capsule or a plurality of tablets or capsules to be taken on a given day.

Another specific embodiment of a kit is a dispenser designed to dispense the daily doses one at a time. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The kits and methods of the present invention may also include, in addition to an estrogen agonist / antagonist as specified herein, one or more additional pharmaceutically active compounds that can be use to treat depression, perimenopausal depression, schizophrenia, anxiety, panic attacks, binge eating, social phobia, or prevent deterioration of cognitive function. Preferably, any additional compound is an estrogen agonist / antagonist or another compound that is useful to treat depression, perimenopausal depression, schizophrenia, anxiety, panic attacks, binge eating, social phobia, or prevent deterioration of cognitive function.

Compounds that are used to treat depression and which can be used in combination with the estrogen agonists / antagonists in the present methods and kits include selective serotonin reuptake inhbitors (SSRIs) such as citalopram (Celexa®), paroxetine (Paxil®), fluoxetine (Prozac®), and sertraline hydrochloride (Zoloft®); tricylclic compounds such as amitriptine (Elvanil®), perphenazine (Etrafon®), chlordiazepoxide and amitriptyline (Limbitrol®), desipramine (Norpramin®), doxepin (Sinequan®), trimipramine (Surmontil®) and protriptyline (Vivactil®); monoamine oxidase inhibitors such as phenelzine (Nardil®) and tranylcypromine ( Parnate®); and other compounds that are used to treat depression such as venlafaxine (Effexor®), mirtazapine (Remeron®), nefazodone (Serzone®) and bupropion (Wellbutrin®).

Compounds that are used to treat anxiety and which can be used in combination with the estrogen agonists / antagonists of the present methods and kits include bezodiazepines such as lorazepam (Ativan®), chlordiazepoxide (Librium®), chlordiazepoxide and amitriptyline (Limbitrol®), clorazepate (Tranxene®), diazepam (Valium®) and alprazolam (Xanax®). Other antianxiety agents that can be used in combination include hydroxyzine (Atarax®), buspirone (BuSpar®), venlafaxin (Effexor®), mephobarbital (Mebaral®), Miltown®, paroxetine (Paxil®), doxepin (Sinequan®), perphenazine and amitriptyline (Triavil®) and hydroxyzine (Vistaril®). Also contemplated for use in combination with an estrogen agonist /antagonist of the present invention are pagoclone, N-{[3-fluoro-4-(2-propylaminoethoxy)]phenyl}-4-oxo-4,5,6,7-tetrahydro-1H-indole-3-carboxamide, or other gamma amino butyric acid (GABA) ligands, particularly GABAₐ agonists.

Compounds that are used to treat panic attacks and which can be used in combination with the estrogen agonists / antagonists in the present methods and kits include clonazepam (Klonopin®), paroxetine (Paxil®), alprazolam (Xanax®) and sertraline hydrochloride (Zoloft®).

Compounds that can also be used in combination with the present estrogen agonsits / antagonists include compounds that are used to treat Alzheimer's disease such as donepezil hydrochloride (Aricept®) and tacrine hydrochloride (Cognex®).

Compounds that can also be used in combination with the present estrogen agonsits / antagonists include compounds that are used to treat schizophrenia such as ziprasidone (Geodon®) or olanzapine (Zyprexa®).

The commercial products noted above may be a particular salt or prodrug of the active compound. For example, Zoloft® is the hydrochloride salt of the active compound sertraline. It is intended that the present invention include salts and prodrugs of active compounds. Thus, when the tradename (e.g., sertraline) or trademark (e.g., Zoloft®) is used, it is intended to mean the active compound or a pharmaceutically acceptable salt or prodrug thereof.

In the combination aspect of the methods and kits of the present invention, the estrogen agonist / antagonist and any additional compounds can be administered in the same dosage form or in separate dosage forms. The dosage forms can be the same (e.g., both tablets) or different. Likewise, the compounds can be administered at the same time or at different times. All variations are intended to be included in the present methods and kits.

The experimental presented below is intended to illustrate particular embodiments of the invention and is not intended to limit the specification, including the claims, in any manner.

All documents cited herein, including patents and patent applications, are hereby incorporated by reference.

### Experimental

The diagnoses and assessment of patients having or at risk of having depression, perimenopausal depression, schizophrenia, anxiety, panic attacks, binge eating, social phobia, or deterioration of cognitive function is well known is the art. Referenced and summarized below are some commonly used protocols for diagnosing, assessing and measuring the progression of these conditions.

### Cognitive Function

To measure cognitive function, objective testing using standardized neuropsychological assessments with established normative values for nonclinical populations can be administered at baseline and at 12 and 24 months. The cognitive assessment measures that can be used include the Modified Mini-Mental State Exam (3MS), Logical Memory, and the Trail making Test (TMT).

### Depression and Perimenopausal Depression

Depression can be assessed with the Center for Epidemiologic Studies Depression Scale, Short Form (CESD-10).

| Measures | |
|---|---|
| Baseline | Center for Epidemiologic Studies Depression Scale, Short Form (CESD-10) Modified Mini-Mental Status Examination (3MS) Logical Memory I and II from Wechsler Memory Scale-Revised (WMS-R). Trail Making Test Parts A & B |
| 12 Months | Center for Epidemiologic Studies Depression Scale, Short Form (CESD-10) Modified Mini-Mental Status Examination (3MS) Logical Memory I and II Trail Making Test Parts A & B |
| 24 Months | Center for Epidemiologic Studies Depression Scale, Short Form (CESD-10) Modified Mini-Mental Status Examination (3MS) Logical Memory I and II Trail Making Test Parts A & B |

### CESD-10

This is a 10-item screening questionnaire for symptoms of depressed mood in older adults. It is derived from the full-length (20-item) version of the Center for Epidemiologic Studies of Depression Scale (CES-D). Similar to the CESD, the CESD-10 demonstrates good reliability and validity in relatively well older adults, and shows good predictive accuracy when compared to the full-length 20-item version (κ = 0.97). Scores may range from 0-30 on the CESD-10. In one large screening sample (N=1542), the mean score was 4.7 (95% Cl: 4.5 - 5.0). A cutoff score of 10 or greater minimized false positives and contained only one false negative (Andresen, et al., 1994).

### Mini-Mental Status Exam (MMSE)

This is an 11-item (30 point) brief screening scale of cognitive status. Scores below 24 are generally recognized as reflecting significant cognitive impairment (Folstein, et al, 1975). Although the test is not sensitive to minor changes in cognitive performance, it provides a useful benchmark of overall cognitive functioning and can identify dramatic changes in cognitive performance that might indicate that the subject has developed new medical or neuropsychiatric conditions. MMSE score ≥ 26 is a conservative inclusion criterion to maximize specificity (i.e., inclusion of those subjects who do not have cognitive impairment at baseline).

### Modified Mini-Mental State Examination (3MS)

This is a revision of the MMSE that includes four additional items (date and place of birth, word fluency, similarities, and delayed recall of words) to sample a wider range of cognitive abilities, an expanded range of scores from 30 to 100 to provide finer discrimination among subjects, and standardized testing and scoring procedures (Teng and Chui, 1987). These modifications were made with the objective of improving the reliability and validity of the MMSE; recent publications support that the 3MS is psychometrically superior to the MMSE (Teng, et al., 1990; Lamarre & Patten, 1991; Grace, et al., 1996; Bravo & Hebert, 1997a; McDowell, et al., 1997). In a non-demented elderly population with an age range 65-69 yrs (N=2098), the mean score and standard deviation (sd) was 90.9 (7.6) (Bravo & Hebert, 1997b).

Empirical data from prospective, epidemiologic studies indicate the magnitude of change in the 3MS score in nondemented populations that could be considered clinically significant. A 5-point or greater decline in 3MS score over 3 yrs in the Cardiovascular Health Study was considered to represent a clinically significant change (Kuller, et al., 1998). In the Canadian Study of Health and Aging, a decline of 10-points (approximately 1sd of the sample 3MS score) over the course of 5 yrs was considered clinically meaningful and correlated with differential rates of dementia and institutionalization (Maxwell, et al., 1999).

### Logical Memory I and II (Wechsler Memory Scale - Revised)

LMI and LMII are standardized tests of verbal memory that examine the ability to recall ideas in two orally presented stories (Wechsler, 1987). LMI is an assessment of immediate recall. LMII measures performance on delayed recall. Based on the standardization sample for the WMS-R, the mean score (and sd) norms for LMI and LMII by age group are: age 55-64: LMI=22.5 (6.3), LMII=18.1 (6.0); age 65-69: LMI=22.0 (7.4), LMII=16.8 (8.1); age 70-74: LMI=20.9 (7.3), LMII 14.7 (9.2) (Wechsler, 1987). In a community dwelling, cognitively unimpaired population with a mean age of 79 yrs (N=234), the mean score (sd) on LMI was 21.3 (6.1) and on LMII was 15.3 (7.6). For those with mild cognitive impairment (MCI; N=76), the mean score on LMI was 12.7 (5.2) and on LMII was 4.2 (5.2) (Petersen, et al., 1999).

### Trail Making Test Parts A & B

This test is an assessment of psychomotor speed and function as well as attention, concentration, sequencing, and mental flexibility. Scores are determined by the time required for completion (max = 30 sec). In a non-demented population with age range 60-69 years (N=61), the mean (sd) for Trails A was 35.8 sec (11.9) and for Trails B was 81.2 sec (38.5) (Spreen & Strauss, 1998).

### References

Andresen, et al. (1994). Screening for depression in well older adults: evaluation of a short form of the CES-D. American Journal of Preventive Medicine, 10:77-84.

Bravo, G. & Hebert, R. (1997a). Reliability of the Modified Mini Mental State Examination in the context of a two-phase community prevalence study. Neuroepidemiology, 16: 141-148.

Bravo, G. & Hebert, R. (1997b). Age- and education-specific reference values for the Mini-mental and Modified Mini-mental State Examinations derived from a nondemented elderly population. International Journal of Geriatric Psychiatry, 12:

### 1008-1018.

Folstein, M., et al. (1975). "Mini-Mental State:" a practical method for grading the cognitive state of patients for the clinician. Journal of Psychiatric Research, 12: 189-198.

Grace, J., Nadler, J., White, D., et al. (1996). Folstein vs. Modified Mini-Mental State Examination in geriatric stroke: stability, validity, and screening utility. Archives of Neurology, 52: 477-484.

Kuller, L., Shemanski, L., Manolio, T., et al. (1998). Relationship between ApoE, MRI findings, and cognitive function in the Cardiovascular Health Study. Stroke, 29: 388-398.

Lamarre, C. and Patten, S. (1991). Evaluation of the Modified Mini Mental State Examination in a general psychiatric population. Canadian Journal of Psychiatry, 36: 507-511.

Maxwell, C., Hogan, D. and Ebly, E. (1999). Calcium-channel blockers and cognitive function in elderly people: results from the Canadian Study of Health and Aging. Canadian Medical Association Journal, 161: 501-506.

McDowell, I., Kristjansson, B., Hill, G., and Hebert, R. (1997). Community screening for dementia: The Mini Mental State Exam (MMSE) and the Modified Mini Mental State Exam (3MS) compared. Journal of Clinical Epidemiology, 50: 377-383.

Mitrushina, M. & Satz, P. (1991). Reliability and validity of the MMSE in neurologically intact elderly. Journal of Clinical Psychology, 47: 537-543.

Petersen, R., et al. (1999). Mild cognitive impairment: clinical characterization and outcome. Archives of Neurology, 56: 303-308.

Spreen, R. & Strauss, E. (1998). A compendium of neuropsychological tests (2^{nd} Ed.), pp. 533-547. New York: Oxford University Press.

Teng, E. & Chui, H. (1987). The modified mini-mental state (3MS) examination. Journal of Clinical Psychiatry, 48: 314-318.

Teng, E., Chui, H., and Gong, H. (1990). Comparisons between the Mini Mental Status Exam (MMSE) and its modified version - the 3MS test. In K. Hasegawa and

A. Homma (Eds.): Psychogeriatrics: Biomedical and Social Advances. Selected Proceedings of the Fourth International Psychogeriatric Association, September 5-8, 1989, Tokyo, Excerpta Medica, Amsterdam, 1990: pp. 189-192.

Wechsler, D. (1987). Wechsler Memory Scale - Revised. San Antonio, TX: The Psychological Corporation.

## Claims

1. The use of an estrogen agonist / antagonist of formula I 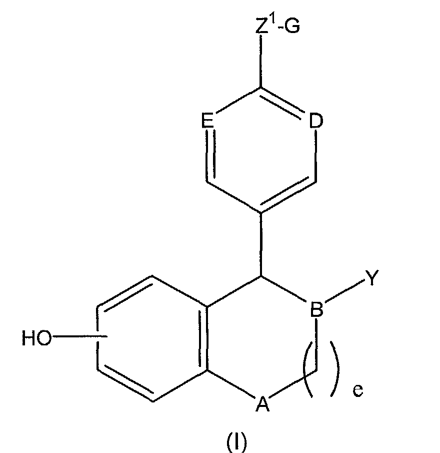 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚW(CH₂)_{q}-;
(b) -O(CH₂)ₚCR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 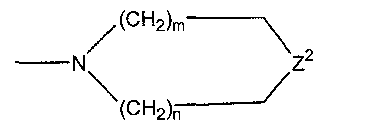 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 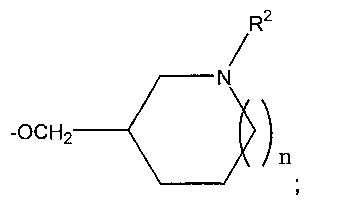
W is
(a) -CH₂-;
(b) -CH=CH-;
(c) -O-;
(d) -NR²-;
(e) -S(O)ₙ-;
(f) 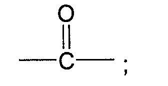
(g) -CR²(OH)-;
(h) -CONR²-;
(i) -NR²CO-;
(j) 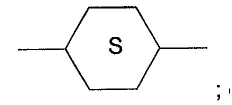 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(l) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, quaternary ammonium salt or prodrug thereof for the preparation of a medicament for the treatment of depression or perimenopausal depression.

2. The use of claim 1 wherein the estrogen agonist / antagonist is a compound of formula (IA) 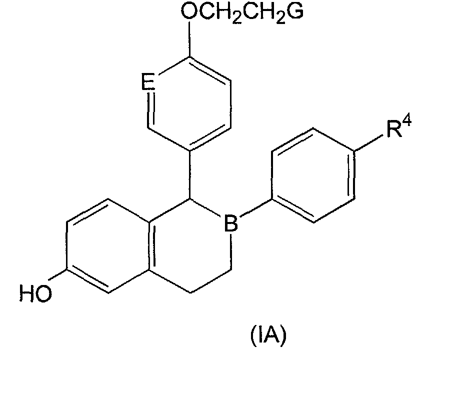 wherein G is 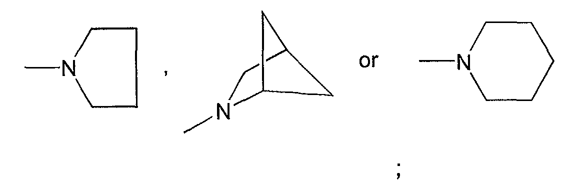 R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

3. The use of claim 1 wherein the estrogen agonist / antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

4. The use of claim 3 wherein the estrogen agonist / antagonist is in the form of a D-tartrate salt.

5. The use of an estrogen agonist / antagonist of Formula I 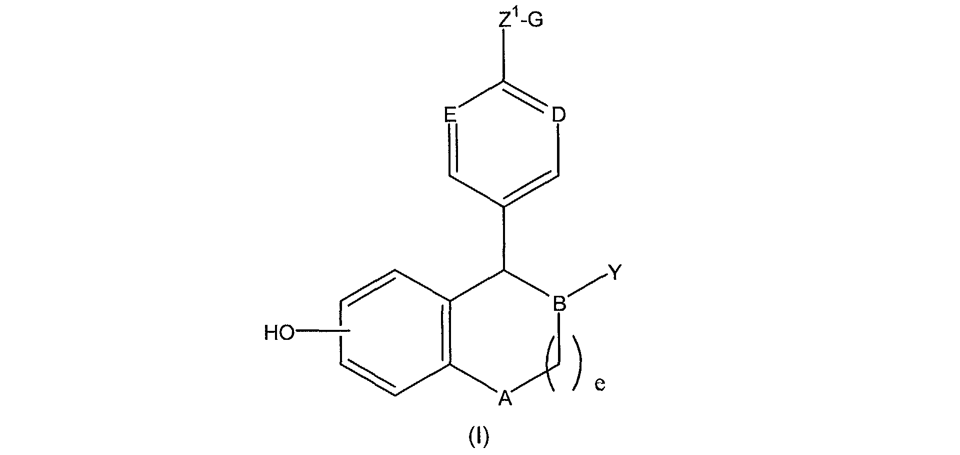 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚW(CH₂)_{q}-;
(b) -O(CH₂)ₚCR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 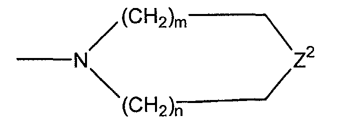 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 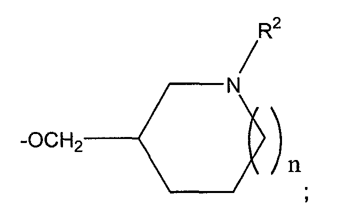
W is
(a) -CH₂-;
(b) -CH=CH-;
(c) -O-;
(d) -NR²-;
(e) -S(O)ₙ-;
(f) 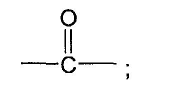
(g) -CR²(OH)-;
(h) -CONR²-;
(i) -NR²CO-;
(j) 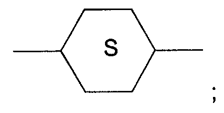 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(I) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, quaternary ammonium salt or prodrug thereof for the preparation of a medicament for the treatment of schizophrenia, anxiety, panic attacks, binge eating or social phobia.

6. The use of claim 5 wherein the estrogen agonist / antagonist is a compound of formula (IA) 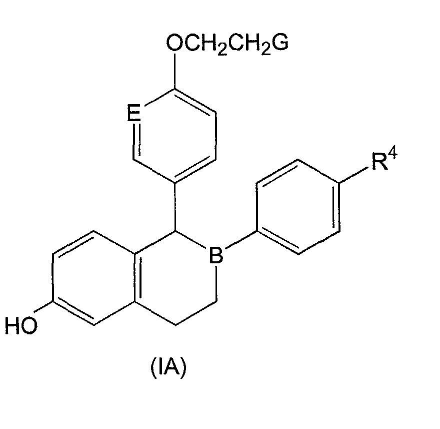 wherein G is 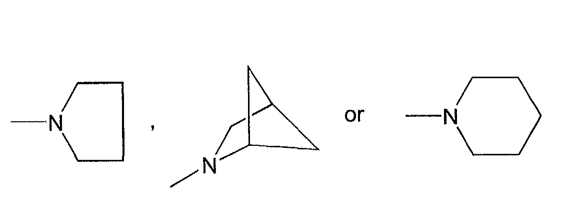 R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

7. The use of claim 5 wherein the estrogen agonist / antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

8. The use of claim 7 wherein the estrogen agonist / antagonist is in the form of a D-tartrate salt.

9. The use of an estrogen agonist / antagonist of Formula I 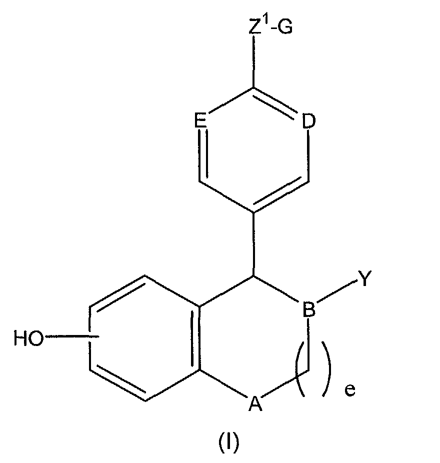 wherein:
A is selected from CH₂ and NR;
B, D and E are independently selected from CH and N;
Y is
(a) phenyl, optionally substituted with 1-3 substituents independently selected from R⁴;
(b) naphthyl, optionally substituted with 1-3 substituents independently selected from R⁴;
(c) C₃-C₈ cycloalkyl, optionally substituted with 1-2 substituents independently selected from R⁴;
(d) C₃-C₈ cycloalkenyl, optionally substituted with 1-2 substituents independently selected from R⁴;
(e) a five membered heterocycle containing up to two heteroatoms selected from the group consisting of -O-, -NR²- and -S(O)ₙ-, optionally substituted with 1-3 substituents independently selected from R⁴;
(f) a six membered heterocycle containing up to two heteroatoms selected from the group consisting of -O-, -NR²- and -S(O)ₙ- optionally substituted with 1-3 substituents independently selected from R⁴; or
(g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
(a) -(CH₂)ₚ W(CH₂)_{q}-;
(b) -O(CH₂)ₚ CR⁵R⁶-;
(c) -O(CH₂)ₚW(CH₂)_{q}-;
(d) -OCHR²CHR³-; or
(e) -SCHR²CHR³-;
G is
(a) -NR⁷R⁸;
(b) 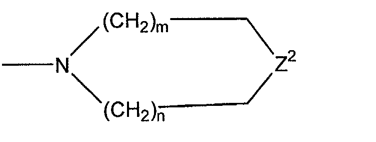 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
(c) a bicyclic amine containing five to twelve carbon atoms, either bridged or fused and optionally substituted with 1-3 substituents independently selected from R⁴; or
Z¹ and G in combination may be 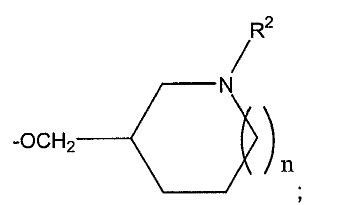
W is
(a) -CH₂-;
(b) -CH=CH-;
(c) -O-;
(d) -NR²-;
(e) -S(O)ₙ-;
(f) 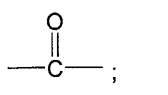
(g) -CR²(OH)-;
(h) -CONR²-;
(i) -NR²CO-;
(j) 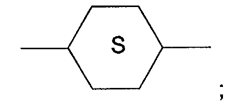 or
(k) -C≡C-;
R is hydrogen or C₁-C₆ alkyl;
R² and R³ are independently
(a) hydrogen; or
(b) C₁-C₄ alkyl;
R⁴ is
(a) hydrogen;
(b) halogen;
(c) C₁-C₆ alkyl;
(d) C₁-C₄ alkoxy;
(e) C₁-C₄ acyloxy;
(f) C₁-C₄ alkylthio;
(g) C₁-C₄ alkylsulfinyl;
(h) C₁-C₄ alkylsulfonyl;
(i) hydroxy (C₁-C₄)alkyl;
(j) aryl (C₁-C₄)alkyl;
(k) -CO₂H;
(I) -CN;
(m) -CONHOR;
(n) -SO₂NHR;
(o) -NH₂;
(p) C₁-C₄ alkylamino;
(q) C₁-C₄ dialkylamino;
(r) -NHSO₂R;
(s) -NO₂;
(t) -aryl; or
(u) -OH;
R⁵ and R⁶ are independently C₁-C₈ alkyl or together form a C₃-C₁₀ carbocyclic ring;
R⁷ and R⁸ are independently
(a) phenyl;
(b) a C₃-C₁₀ carbocyclic ring, saturated or unsaturated;
(c) a C₃-C₁₀ heterocyclic ring containing up to two heteroatoms, selected from -O-, -N- and -S-;
(d) H;
(e) C₁-C₆ alkyl; or
(f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
or an optical or geometric isomer thereof; or a pharmaceutically acceptable salt, N-oxide, ester, quaternary ammonium salt or prodrug thereof for the preparation of a medicament for preventing the deterioration of cognitive function.

10. The use of claim 9 wherein the estrogen agonist / antagonist is a compound of formula (IA) 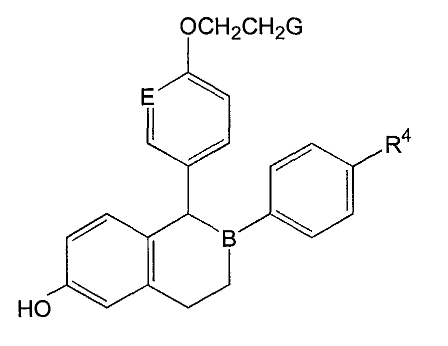 wherein G is 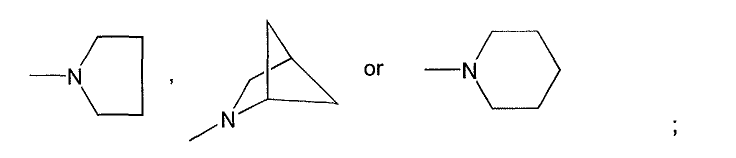 R⁴ is H, OH, F, or Cl; and B and E are independently selected from CH and N or an optical or geometric isomer thereof; or a pharmaceutically acceptable salt, N-oxide, ester, quaternary ammonium salt, or a prodrug thereof.

11. The use of claim 9 wherein the estrogen agonist / antagonist is (-)-cis-6-phenyl-5-[4-(2-pyrrolidin-1-yl-ethoxy)-phenyl]-5,6,7,8-tetrahydro-naphthalene-2-ol or an optical or geometric isomer thereof; a pharmaceutically acceptable salt, N-oxide, ester, quaternary ammonium salt, or a prodrug thereof.

12. The use of claim 11 wherein the estrogen agonist / antagonist is in the form of a D-tartrate salt.
